**Europäisches Patentamt**

**European Patent Office**

**Office européen des brevets**

(19)

(11) Veröffentlichungsnummer : **0 308 436 B1**

(12) # EUROPÄISCHE PATENTSCHRIFT

(45) Veröffentlichungstag der Patentschrift :
**01.07.92 Patentblatt 92/27**

(21) Anmeldenummer : **88901939.4**

(22) Anmeldetag : **02.03.88**

(86) Internationale Anmeldenummer :
**PCT/HU88/00009**

(87) Internationale Veröffentlichungsnummer :
**WO 88/07364 06.10.88 Gazette 88/22**

(51) Int. Cl.⁵ : **A61K 9/06,** A61K 9/70,
A61K 31/125, A61K 31/65,
A61K 31/71, A61K 33/18,
A61K 7/40, A61K 7/48,
A61L 15/16

(54) **NEUE HEILSALBE, VERFAHREN ZU IHRER HERSTELLUNG, UNTER VERWENDUNG DIESER HEILSALBE HERGESTELLTE APPLIKATIONSFORMEN SOWIE DEREN HERSTELLUNG.**

(30) Priorität : **01.04.87 HU 139487**

(43) Veröffentlichungstag der Anmeldung :
**29.03.89 Patentblatt 89/13**

(45) Bekanntmachung des Hinweises auf die
Patenterteilung :
**01.07.92 Patentblatt 92/27**

(84) Benannte Vertragsstaaten :
**AT BE CH DE FR GB IT LI LU NL SE**

(56) Entgegenhaltungen :
**EP-A- 60 553
EP-A- 124 774
EP-B- 1 871
DE-A- 2 514 873
DE-A- 3 341 770
GB-A- 1 502 680
US-A- 4 122 158
HANDBUCH DER KOSMETIKA UND RIECHSTOFFE,
vol. III, 1973, Dr.Alfred Hüttig Verlag GmbH, Heidelberg (DE); pp. 551-753
ACNE PRODUCTS; R.E.HOPPONEN, pp. 321-323**

(73) Patentinhaber : **BIOGAL GYOGYSZERGYAR**
**Pallagi ut 13**
**H-4042 Debrecen (HU)**

(72) Erfinder : **GROSKA, Lászlo**
**Jozsef A. u. 84**
**H-1084 Budapest (HU)**
Erfinder : **KOCSAR, Barna**
**Illyés Gy. u. 68**
**H-4032-Debrecen (HU)**

Erfinder : **TAMAS, András**
**Gárdonyi G. u. 39**
**H-4032 Debrecen (HU)**
Erfinder : **GYÖRGYNE VAGO, Magdolna**
**István u. 63**
**H-4031 Debrecen (HU)**
Erfinder : **MEDGYESI, Ferencné**
**Gárdonyi G. u. 48**
**H-4032 Debrecen (HU)**
Erfinder : **BACSA, György**
**Csao ut. 90**
**H-4029 Debrecen (HU)**
Erfinder : **LORANT, Adorján**
**Károlyi M. u. 1**
**H-4032 Debrecen (HU)**
Erfinder : **JANCSO, Sándor**
**Kardos u. 28**
**H-4028 Debrecen (HU)**
Erfinder : **TAKACS, Erzsébet**
**Böszörményi ut 119**
**H-4032 Debrecen (HU)**
Erfinder : **KOVACS, István**
**Csap ut. 86**
**H-4029 Debrecen (HU)**
Erfinder : **KOBEZDANE FUXREITER, Margit**
**Burgundia u. 10**
**H-4024 Debrecen (HU)**
Erfinder : **SBESTYEN, Pál**
**Darabos u. 12**
**H-4026 Debrecen (HU)**
Erfinder : **GOMBOS, Zoltán**
**Simonyi u. 30/b**
**H-4028 Debrecen (HU)**
Erfinder : **FARAGO, Tamás**
**Mártirok utja 56**
**H-1027 Budapest (HU)**

(74) Vertreter : **Beszédes, Stephan G., Dr. Patentanwalt**
**Münchener Strasse 80a Postfach 1168**
**W-8060 Dachau (DE)**

EP 0 308 436 B1

**Beschreibung**

Die Erfindung betrifft eine neue, im wesentlichen wasserfreie Heilsalbe, ein Verfahren zu deren Herstellung, die unter Verwendung dieser Heilsalbe hergestellten Applikationsformen sowie deren Herstellung. Die erfindungsgemäße Salbe beziehungsweise die unter ihrer Verwendung hergestellten Applikationsformen (Pflaster, imprägnierte Mullbinden, Gaze usw.) werden zur Behandlung beziehungsweise Heilung von Verletzungen verwendet, bei denen die Oberhaut oder die gesamte Haut zerstört ist. Derartige Wunden sind zum Beispiel Verbrennungen zweiten und dritten Grades, durch Abschürfungen, Geschwüre usw. hervorgerufene Löcher in der Haut, tiefe Verbrennungen sowie weitere Destruktionen der Haut, die auch Hauttransplantationen erforderlich machen können.

Brandwunden, insbesondere wenn sie große Flächen des Körpers bedecken beziehungsweise wenn sie zweiten oder dritten Grades sind, können nicht lediglich als auf die Haut lokalisierter krankhafter Prozeß betrachtet werden; vielmehr handelt es sich bei ihnen um einen den gesamten Organismus in Mitleidenschaft ziehenden, in schweren Fällen lebensgefährlichen Krankheitsprozeß. Durch die hohe Temperatur, ferner durch die Wirkung starker Bestrahlung oder chemischer Agentien treten endogene, vasoaktive Stoffe (Histamin, Kinine) aus den Geweben der hautlosen Stellen aus, und es kommt zu einer Erhöhung der Durchlaßfähigkeit der Kapillargefäße, Plasma tritt aus dem Gefäßsystem aus, die im Kreislauf geführte Blutmenge wird geringer. Wenn das Gefäßsystem nicht binnen kurzer Frist aufgefüllt wird, so treten hauptsächlich im Herzen und in der Niere irreversible Veränderungen ein, die den Tod des Kranken zur Folge haben können (Arch. Osp. Mare 20, 432-444, 1968).

Der zweite Abschnitt der Verbrennungskrankheit ist eine septisch-toxische Phase, die von den sich auch den abgestorbenen Teilen der Haut vermehrenden Bakterien und Pilzen sowie den von der verletzten Stelle resorbierten giftigen Substanzen hervorgerufen wird.

Die erste Aufgabe bei der Behandlung von Verbrennungen ist die Beseitigung des Schockes, dann die Vermeidung oder doch wenigstens Verkürzung des septisch-toxischen Abschnitts und schließlich der Ersatz der verbrannten Gewebe, Korrektion der einen kosmetischen oder funktionellen Nachteil bedeutenden Narben.

Nach der Beseitigung des Schocks kann mit der Behandlung der Wunde, mit dem Entfernen der abgestorbenen Gewebe begonnen werden. Der Zweck der lokalen Behandlung besteht (da die physiologischen und Schutzfunktionen der Haut ausgefallen sind) darin, die Wunde vor physischen Schäden, vor dem Austrocknen und vor Flüssigkeits- und Elektrolytverlust sowie vor Infektionen zu schützen und den Heilungsprozeß zu fördern.

Gegenwärtig werden zur Wundbehandlung hautbildende Salben (Perubalsam, Mikulitzsalbe, Panthenolol), mit dem Pinsel auftragbare bakterizide Mittel verwendet. Die Anwendung der Salben erfolgt entweder, indem die zerstörte Hautfläche mit der Salbe bestrichen und im weiteren eine offene oder geschlossene Behandlung durchgeführt wird, oder die Salbe wird auf Textilien aufgetragen und als Pflaster verwendet. Derartige Pflaster haben jedoch den Nachteil, daß auch bei häufigem Verbandswechsel die Wunde zu lange mit der von ihr ausgeschiedenen Flüssigkeit in Berührung steht und dadurch eine oberflächliche Verbrennung zu einer auch die tieferen Hautschichten in Mitleidenschaft ziehenden Tiefenverbrennung werden kann.

Zur Herstellung antiseptischer, chemotherapeutischer Salben sind zahlreiche Versuche unternommen worden. Gemäß der US-PS 4 401 651 wird eine antimikrobiell wirkende Salbe in dicker Schicht auf einen Tampon aufgestrichen, und der Tampon wird mit der eingestrichenen Seite auf die Wunde aufgelegt. Dabei bedeckt der Tampon die verletzte Oberfläche ganz.

In der US-PS 4 301 145 ist eine antiseptische Krem beschrieben, die PVP-$J_2$-Komplex (Polyvinylpyrrolidon-Jod-Komplex), Natriumcitrat, Glycerin, Stearin oder Cetylalkohol, Konservierungsmittel, Emulgiermittel und höchstens 85 % Paraffinderivat als Kremgrundlage enthält. Gemäß der Beschreibung verfügt die Krem über bakterizide, fungizide und virizide Wirkung.

In der britischen Patentschrift Nr. 673 587 ist eine "trockene" Salbe beschrieben, die auch zusammen mit therapeutisch wirksamen Agentien verwendet werden kann, die gegen Wasser empfindlich sind. Die Salbe enthält als Wirkstoff Antibiotika, zum Beispiel Bacitracin oder das Kaliumsalz von Penicillin-G. Die Salbengrundlage enthält unter anderem Carboxymethylcellulose, als Netzmittel Natriumdodecylsulfat, als quellfähiges natürliches Mineral Bentonit, ferner Zucker wie ß-Lactose und außerdem wasserfreie Citronensäure.

Zur Behandlung von Brandwunden und Transplantationsgebieten wird in US-PS 4 303 066 eine eigenartige Kombination von Therapie und Abdecken der Wunde vorgeschlagen. Gemäß der Beschreibung ist ein völlig abschließender, mechanisch widerstandsfähiger, durchscheinender und nicht klebender Verband auf jeden Fall wünschenswert. Die Bildung stabiler Polymerfilme in situ aus organischem Trägermaterial und einem hydrophilen Polymer dauert häufig bis zu einer Stunde. Das ist für den Patienten sehr unangenehm, denn er muß die ganze Zeit über unbeweglich bleiben, und das verwendete Lösungsmittel kann Schmerzen verursachen.

Der pH-Wert der Wunde und ihrer unmittelbaren Umgebung ist im allgemeinen niedriger (saurer) als der der normalen Haut, und dieser Umstand verhindert die Anwendung zahlreicher Agentien.

Weitere Anwendungsschranken ergeben sich durch die Anwesenheit von Wasser. Die im allgemeinen zur Wundbehandlung verwendeten Salben und Krems, die in beträchtlichem Maße Wasser enthalten, zum Beispiel o/w- und w/o-Emulsionen, entziehen der Wunde das Wasder, trocknen sie auf diese Weise aus und verursachen unerwünschte Narbenbildung; die Heilung der Wunde wird verzögert.

Wasserhaltige Präparate und Emulsionen ermöglichen die Anwendung zahlreicher Wirkstoffe nur beschränkt oder überhaupt nicht.

Die Verwendung von Jod, insbesondere von komplex gebundenem Jod zur Herstellung antiseptischer Bedingungen ist seit langem bekannt. Aus den Komplexen wird das Jod sehr langsam freigesetzt und übt am Ort der Behandlung eine bakterizide und fungizide Wirkung aus. In Präparaten dieser Art beträgt der Jodgehalt im allgemeinen höchstens 2 % (J. Trauma 25, 3, 247-249, 1985).

Die beste bakterizide, fungizide und virizide Wirkung kann erfahrungsgemäß mit Jod erzielt werden. Für die ausgezeichnete antimikrobielle Wirkung spricht auch, daß bei der Anwendung als Komplex in der Umgebung eine niedrige, aber ständige Konzentration an Jod völlig antiseptische Bedingungen gewährleistet.

Die zur Behandlung der üblichen Hautschäden klinisch allgemein angewendeten bekannten Salben werden häufig auf Gazeträger aufgebracht. Die Gazeträger werden im allgemeinen heißluftsterilisiert und dann zum Abdecken der Wunde verwendet, Gaze wird sowohl zum Behandeln offener wie auch zum Behandeln geschlossener Wunden häufig verwendet. Beim Imprägnieren der Gazeträger und anderer Textilträger treten häufig schwer lösbare technische Probleme auf. Zum Beispiel kann sich bei der Verwendung von Mikulitzsalbe Zinkoxyd ausscheiden, und bei der Heißluftbehandlung kann es zur Ausscheidung weiterer Stoffe kommen. Die Salben enthalten auch häufig wärmeempfindliche Wirkstoffe, die durch die Wärmesterilisation geschädigt werden können und dabei möglicherweise sogar die Wundheilung verzögernde Zersetzungsprodukte bilden.

Schließlich ist bei der Verwendung der bekannten Präparate, insbesondere des Perubalsams, in einem großen Teil der behandelten Fälle das Auftreten von Allergien zu verzeichnen.

Gegenwärtig werden beträchtliche Mengen an Wollwachs verwendet, zum Beispiel in dem Präparat "Grassolind" (Paul Hartmann AG, BRD), das 76 % weiße Vaseline, 22 % Wollwachs und 2 % flüssiges Paraffin enthält. Die mit dem Präparat imprägnierten Textilien dienen zum Schutz der Wunden und dem Aufsaugen von Wundflüssigkeit. Durch die beträchtliche Konzentration an Wollwachs kommt es häufig zur Ausbildung von Kontaktallergien (ungarische Patentschrift Nr. 187 413).

Ziel der Erfindung war es, eine Salbe bereitzustellen, die infolge ihrer Zusammensetzung keine Allergie, keine Hautirritierung verursacht, gut zur Imprägnierung von in der Therapie verwendbaren Textilien geeignet ist und beim Sterilisieren nicht in ihre Phasen zerfällt. Bei Anwendung der Salbe werden die potentiellen Regenerationseigenschaften der Wunde optimal genutzt. Die Salbe schafft sowohl bei geschlossener wie auch bei offener Behandlung der Wunde die besten Bedingungen für eine Autoregeneration des Gewebes.

Gegenstand der Erfindung ist demnach eine Heilsalbe, insbesondere zur Behandlung von Verbrennungen. Für die erfindungsgemäße Heilsalbe ist kennzeichnend, daß sie

12 - 25    Masse% Wachs,
60 - 88    Masse% pharmakologisch verträgliches Öl,
1 - 15     Masse% einer kühlenden ätherischen Substanz und gegebenenfalls
max. 5     Masse% mit den übrigen Komponenten verträgliche Wirkstoffe
enthält.

Innerhalb des angegebenen Wachsgehaltes ist das Wachs bevorzugt zu 15-20 Masse% enthalten; besonders geeignet sind Bienenwachs und/oder Wollwachs, besonders bevorzugt ist das weiße Bienenwachs. Das pharmakologisch verträgliche Öl ist zweckmäßig in einer Menge von 68-78 Masse% enthalten; es handelt sich bevorzugt um Paraffinöl, Mygliol, Sonnenblumenöl, Ricinusöl oder deren Gemische. Die kühlende ätherische Substanz ist vorzugsweise in einer Menge von 2-9 Masse% enthalten, sie wird vorzugsweise von Campher und/oder Menthol gebildet. Als Wirkstoffe schließlich kommen vor allem Antiseptika und Chemotherapeutika in Frage. Als Antiseptikum kommen Jod oder Jod-komplexe, wie zum Beispiel der Polyvinylpyrrolidon-Jod-Komplex, als Chemotherapeutika Antibiotika wie Neomycin, Tobramycin, Erythromycin, Oxytetracyclin in Frage. Die Wirkstoffe machen bevorzugt 0,5-2 Masse% der Salbe aus.

Die Erfindung betrifft ferner ein Verfahren zur Herstellung vor Heilsalbe zur lokalen Anwendung. Das erfindungsgemäße Verfahren ist dadurch gekennzeichnet, daß aus 12-25 Masse% Wachs, 60-88 Masse% pharmakologisch verträglichem Öl, 1-15 Masse% einer Kühlenden ätherischen Substanz und gegebenenfalls max. 5 Masse% Widrkstoff durch Erwärmen und Homogenisieren eine Schmelze hergestellt und diese abgekühlt wird.

Die Erfindung betrifft ferner lokale Applikationsformen zur Behandlung von Hautschäden, insbesondere Verbrennungen. Für diese lokalen Applikationsformen ist kennzeichnend, daß sie auf Textilträgern wie Mullbinden, Gaze, Leinen oder Folie aufgestrichen die oben beschriebene Heilsalbe enthalten.

Schließlich betrifft die Erfindung ein Verfahren zur Herstellung der lokalen Applikationsformen. Das Verfahren ist dadurch gekennzeichnet, daß die erwähnte Heilsalbe auf textile Träger wie Mull, Gaze, Leinen, oder auf Folie aufgestrichen wird, wobei auf die Masse des Trägers bezogen das 0,2-10fache, vorzugsweise das 1-3fache an Salbe zur Verwendung gelangt, und das bestrichene Trägermaterial gewünschtenfalls sterilisiert wird.

Zur Ausarbeitung einer optimalen Zusammensetzung der Salbe wurden zahlreiche Versuche unternommen. Zu Beginn wurde weißes Bienenwachs und Paraffinöl in einem Verhältnis von 1:1-2 verwendet. Die auf diese Weise erhaltene Salbe war jedoch bei Raumtemperatur zu hart, und auch die imprägnierten Textilien erhärteten zu stark. Es konnte nicht gewährleistet werden, daß nur die Fäden durchtränkt wurden, die Zwischenräume des lockeren Gewebes jedoch freibleiben. Deshalb wurde ein Wachs-Öl-Verhaltnis von 1:3-6 gewählt (insbesondere 1:4). Auch durch Zusatz der kühlenden ätherischen Substanz wird der Erstarrungspunkt stark gesenkt.

Der Zusatz von hydrophilen Stoffen wie Polyoxäthenderivaten zu dem weißen Bienenwachs ermöglicht es zwar, daß bei etwa 60 °C eine homogene Schmelze entsteht, jedoch beim Abkühlen scheidet sich das Wachs wieder aus.

Als pharmakologisch verträgliches Öl kann auch Öl pflanzlichen oder tierischen Ursprungs verwendet werden. In den untersuchten Ölen lösen sich die verwendeten kühlenden ätherischen Substanzen (Campher, Menthol) gut, und auch die übrigen Wirkstoffe, zum Beispiel das Jod oder die Jodkomplexe, verteilen sich gut in der Salbengrundlage.

Als Wachs wird bevorzugt weißes Bienenwachs (Cera alba) verwendet. Ein Ersatz durch Wollwachs kommt mur in Frage, wenn sich ein gewünschter Wirkstoff auf andere Weise nicht einarbeiten läßt.

Zur Herstellung der erfindungsgemäßen Salbe wird das Wachs zusammen mit einem Teil des pharmakologisch verträglichen Öls geschmolzen. Wenn zur Herstellung der Salbe alkoholische Lösungen verwendet werden (zum Beispiel alkoholische Jodlösung), so wird die kühlende Substanz in dieser gelöst, die Lösung mit dem verbliebenen Teil des Öles vermischt und dann, vorzugsweise auf dem Wasserbad, der auf etwa 70 °C erwärmten Wachsschmelze zugesetzt. Falls keine alkoholischen Lösungen verwendet werden, kann die kühlende Substanz mit einem Teil des Öles vermischt und in dieser Form der Wachsschmelze zugesetzt werden. Das Gemisch wird bis zur völligen Homogenität bei der genannten Temperatur gerührt. Das Rühren wird bis zum völligen Abkühlen fortgesetzt.

Die ebenfalls zum Gegenstand der Erfindung gehörenden Applikationsformen werden hergestellt, indem geeignete textile Träger oder Folien mit der erfindungsgemäßen Salbe imprägniert oder bestrichen werden. Es kann vorteilhaft sein, die Salbe zu diesem Zweck etwas zu erwärmen. Besonders vorteilhaft ist als Trägermaterial locker gewebter Mull; die imprägnierten Fäden bilden ein "antiseptisches Gitter", das einesteils die Niederlassung und Vermehrung von Bakterien und Pilzen nicht zuläßt, zum anderen jedoch der Luft freien Zutritt zur Wundoberfläche gestattet und dadurch die Heilung beschleunigt. Ein weiterer Vorteil des antiseptischen Gitters besteht darin, daß Ausscheidungen der Wunde (Brandwunden nässen meistens) durch einfaches Abtupfen entfernt werden können. Die Salbe kann ferner zur Imprägnierung des Mullkissens von Heftpflastern (Leukoplast) verwendet werden. Nach dem Aufkleben des Pflasters hält das Kissen die Wunde steril, verringert durch die kühlende Substanz den Juckreiz und fördert die Heilung.

Die erfindungsgemäßen Applikationsformen, insbesondere imprägnierte Textilien sind atraumatisch, enthalten kein Wasser und kleben deshalb nicht an der Wunde fest. Ihr Wechsel schädigt die neu entstehenden Gewebe nicht und ist völlig schmerzlos. Vor Aufbringen des neuen Pflasters (Verbandes) können Antiseptika lokal angewendet werden.

Die Wirkung der erfindungsgemäßen Salbe und der mit dieser imprägnierten Applikationsformen wurde an einem Wundheilungsmodell im Tierversuch erprobt (Linsky C.B., Rovee, D.T.: The influence of local environment on the course of wound healing in the guinea pig; Int. Symp. Wound Healing, Rotterdam, S. 211-213, Foundation International Cooperation in the medical Sciences, Montreaux 1974). Als Versuchstiere dienten Meerschweinchen von 270-500 Gewicht (die Hälfte männlichen, die andere Hälfte weiblichen Geschlechts). Auf dem Rücken der Tiere wurde eine 5x5 cm große Fläche mit der Schere, dann mit dem elektrischen Rasierapparat enthaart und schließlich mit Alkohol sterilisiert. Auf dieser Fläche wird mit einem kreisförmigen Messer von 2,3 cm Durchmesser ein die gesamte Dicke von Oberhaut und Unterhaut umfassender Hautausschnitt vorgenommen. Die Wunden werden auf folgende Weise versorgt. 10x10 cm große Gazestücke werden mit der gemäß Beispiel 3 hergestellten salbe imprägniert, wobei pro Gazestück 1-1,8 g Salbe Verwendung finden. Diese Menge ist ausreichend, die Fäden der Gaze zu durchtränken, die Zwischenräume bleiben jedoch frei. Diese imprägnierten Gazestücke werden auf die Wunde aufgelegt, dann mit steriler Gaze festgebunden und schließlich der Verband mit Leukoplast fixiert. Der Verband wurde über 8 Tage täglich gewechselt. Am Ende des Versuches wurde der Grad der Heilung durch die meßbare Kontraktion der Wunde charakterisiert. Die erhaltenen Werte sowie die von Kontrolle und Referenzpräparaten sind in der Tabelle 1 zusammengefaßt.

Die Kontraktion der Wunde war im Falle des Präparates gemäß Beispiel 3 signifikant besser als im Fall der Kontrolle und der Referenzpräparate. Besonders fiel die außerordentlich schnelle Entwicklung des Granulationsgewebes auf, die Wundheilung verlief wesentlich schneller.

Besonders interessant ist der Vergleich mit dem Präparat Braunovidon[R]; diese Referenzsubstanz enthält Polyvinylpyrrolidon-Jod-Komplex, und ihr Gehalt an freiem Jod stimmt mit dem Jodgehalt der erfindungsgemäßen Salbe überein. Die Braunovidon[R]-salbe wurde ebenso wie die erfindungsgemäße Salbe auf Gaze aufgestrichen, und die Versuchsbedingungen waren die gleichen. Die Tabelle zeigt einen signifikanten Unterschied in der Wirkung.

An 12 DHP-Meerschweinchen von 250-300 g Gewicht wurden sensibilisierende Wirkungsuntersuchungen durchgeführt. Dabei wurde der offene Epikutantest (Organization for Economic Cooperation and Development Guidelines /1981/) angewendet. Die Tiere wurden auf der einen Seite mit dem elektrischen Rasierapparat enthaart und dann über 10 Tage lang täglich einmal mit der Salbe gemäß Beispiel 3 eingerieben, bis sich die Haut rötete. Die Kontrolltiere wurden trocken gerieben. Am Ende des Versuches konnte beobachtet werden, daß sich ein Erythema weder in der primären Irritationsphase noch in der anschließenden Sensibilisationsphase ausbildete.

Versuche zur Hautirritation wurden auch an 6 Stück Neuseeländer Kaninchen von 2-3 kg Gewicht vorgenommen (Versuchseinstellung: Code of Federal Regulations, Title 16, Section 1500.41). 24 Stunden vor Beginn des Versuches wurden die Tiere auf dem Rücken mit einer Schere und anschließend mit 20 %iger Natriumsulfidlösung enthaart. Nach Waschen mit Leitungswasser wurde mit einer 1 %igen Essiglösung das anhaftende Natriumsulfid neutralisiert. Die Salbe gemäß Beispiel 3 wurde auf ein Gazestück gestrichen und auf die Haut aufgebracht. Die Gaze wurde mit Cellophan abgedeckt und mit einem Pflaster befestigt. Nach 24 Stunden wurde der Verband abgenommen, die Haut wurde von anhaftenden Salbenresten befreit. Aus dem Durchschnitt der nach 24 und nach 72 Stunden vorgenommenen Bonitierungen wurde der primäre Irritationsindex gebildet. Die Salbe gemäß Beispiel 3 wirkt nicht irritierend, ihr primärer Irritationsindex ist gleich Null.

Versuche zur Irritierung des Auges wurden an 11 Stück Neuseeländer Kaninchen von 1,5-1,7 kg Gewicht vorgenommen (Versuchseinstellung: siehe oben, jedoch Section 1500.42). 0,1 ml der Salbe gemäß Beispiel 3 wurde den Tieren mit einer Mikropipette auf die Mitte der Hornhaut des rechten Auges aufgetragen. Als Kontrolle wurde das linke Auge betrachtet. Eine Stunde nach dem Auftragen der Salbe wurde eine verstärkte Tränenbildung sowie eine verstärkte Durchblutung der Conjunctiva beobachtet, diese Symptome gingen jedoch innerhalb von 24 Stunden zurück. Der (gemäß der angegebenen Literatur) gemessene Draize-Index betrug für die Salbe gemäß Beispiel 3 0,7, was bedeutet, daß die Salbe schwach irritierend auf die Augen wirkt.

Untersuchungen zur Hauttoxizität wurden an 20 Stück Wistar-Ratten von 200-270 g Gewicht (10 Stück waren männlichen, 10 Stück weiblichen Geschlechts) vorgenommen. Die Versuche wurden gemäß Organization for Economic Corporation and Development Guidelines /1981/ vorgenommen. 40-50 % der Körperfläche der Versuchstiere wurde 24 Stunden vor Beginn des Versuches mit der Schere und anschließend mit 20 %iger Natriumsulfidlösung enthaart, dann mit Wasser gewaschen und schließlich mit 1 %iger Essigsäure neutralisiert. Die Salbe gemäß Beispiel 3 wurde in einer Dosis von 5 g/kg mit Filterpapier auf die enthaarte Fläche aufgebracht, mit Cellophan abgedeckt und mit einem Pflaster fixiert Nach 24 Stunden wurden die Verbände entfernt, die Salbenreste wurden abgewaschen. Während der gesamten Versuchsdauer zeigten die behandelten Tiere im Zustand ihrer Haut, im Verhalten und der Gewichtszunahme keinen Unterschied zur unbehandelten Kontrolle. Der dermale $LD_{50}$-Wert der Salbe gemäß Beispiel 3 ist größer als 5000 mg/kg Körpergewicht.

Die Erfindung wird an Hand der folgenden Beispiele näher erläutert.

## Beispiel 1

| | |
|---|---|
| Weißes Bienenwachs | 17.8 g |
| Paraffinöl | 71,2 g |
| Campher | 9,0 g |
| alkoholische Jodlösung | 2,0 g |
| (5 %ig) | 100,0 g |

Der Campher wird in der alkoholischen Jodlösung gelöst und zu der Lösung ein Teil des Paraffinöls gegeben. Das weiße Bienenwachs wird zusammen mit dem restlichen Paraffinöl auf dem Wasserbad bei 68 °C geschmolzen und dann mit dem campherhaltigen Gemisch homogenisert. Die Schmelze wird bis zum Auskühlen gerührt. Die erhaltene Salbe ist von gelber Farbe und hat einen charakteristischen Camphergeruch.

Erstarrungspunkt: 41-43 °C.

### Beispiel 2

| | |
|---|---:|
| Weißes Bienenwachs | 18,0 g |
| Paraffinöl | 72,0 g |
| Campher | 9,0 g |
| PVP-$J_2$, mikronisiert | 1,0 g |
| | 100,0 g |

Der Campher wird mit dem mikronisierten (feinverteilten) Polyvinylpyrrolidon-Jod-Komplex vermischt und zu dem Gemisch ein Teil des Paraffinöls gegeben. Das weiße Bienenwachs wird mit dem restlichen Paraffinöl zu einer Schmelze erwärmt. Im folgenden wird wie in Beispiel 1 vorgegangen. Die erhaltene Salbe ist von gelblich-brauner Farbe und hat einen charakteristischen Camphergeruch. Erstarrungspunkt: 42-43 °C.

### Beispiel 3

| | |
|---|---:|
| Weißes Bienenwachs | 16,0 g |
| Paraffinöl | 80,0 g |
| Campher | 3,0 g |
| PVP-$J_2$, mikronisiert | 1,0 g |
| | 100,0 g |

Die Salbe wird auf die im Beispiel 2 beschriebene Weise hergestellt. Erstarrungspunkt: 43-44 °C.

### Beispiel 4

| | |
|---|---:|
| Weißes Bienenwachs | 16,0 g |
| Paraffinöl | 81,0 G |
| Menthol | 2,0 g |
| PVP-$J_2$, mikronisiert | 1,0 g |
| | 100,0 g |

Die Salbe wird auf die im Beispiel 2 beschriebene Weise zubereitet. Erstarrungspunkt: 42 °C

### Beispiel 5

| | |
|---|---:|
| Weißes Bienenwachs | 18,0 g |
| Paraffinöl | 73,0 g |
| Campher | 9,0 g |
| | 100,0 g |

Der Campher wird in einem Teil des Paraffinöls gelöst und die Lösung zu der aus dem weißen Bienenwachs und dem Rest des Paraffinöls bereiteten Schmelze gegeben. Die Schmelze wird bis zum Auskühlen gerührt. Die erhaltene Salbe ist weiß und riecht charakteristisch nach Campher. Erstarrungspunkt: 43-44 °C.

Beispiel 6

| Weißes Bienenwachs | 16,0 g |
| --- | --- |
| Paraffinöl | 82,0 g |
| Menthol | 2,0 g |
| | 100,0 g |

Die Salbe wird auf die im Beispiel 5 beschriebene Weise zubereitet. Sie ist weiss und riecht charakteristisch nach Menthol.

Beispiel 7

| Weißes Bienenwachs | 18,0 g |
| --- | --- |
| Rizinusöl | 77,0 g |
| Menthol | 3,0 g |
| alkoholische Jodlösung | 2,0 g |

Die Salbe wird gemäß Beispiel 1 hergestellt. Erstarrungspunkt: 57 °C.

Beispiel 8

| Weißes Bienenwachs | 18,0 g |
| --- | --- |
| Inwitor 742 (Ölgemisch, Dynamit Nobel) | 78,0 g |
| Campher | 3,0 g |
| PVP-$J_2$, mikronisiert | 1,0 g |

Die Salbe wird gemäß Beispiel 2 bereitet. Erstarrungspunkt: 53 °C.

Beispiel 9

| Weißes Bienenwachs | 18,0 g |
| --- | --- |
| Inwitor 742 | 78,0 g |
| Menthol | 3,0 g |
| PVP-$J_2$, mikronisiert | 1,0 g |
| | 100,0 g |

Die Salbe wird auf die im Beispiel 2 beschriebene Weise zubereitet. Erstarrungspunkt: 51 °C.

Beispiel 10

| Weißes Bienenwachs | 16,0 g |
| --- | --- |
| Paraffinöl | 76,0 g |
| Wollwachs | 4,5 g |
| Campher | 3,0 g |
| Erythromycin (Base) | 0,5 g |
| | 100,0 g |

Das eine Teilchengröße von unter 160 µm aufweisende Erythromycin wird mit dem Wollwachs verrieben. Ein Teil des Paraffinöls wird mit dem weißen Bienenwachs zusammen zu einer Schmelze verarbeitet. In dem verbleibenden Paraffinöl wird der Campher gelöst. Schließlich werden alle Bestandteile zu einer homogenen Schmelze geformt. Die Schmelze wird bis zum Abkühlen gerührt. Erstarrungspunkt: 40-41 °C.

### Beispiel 11

| | |
|---|---|
| Weißes Bienenwachs | 16,0 g |
| Sonnenblumenöl | 76,0 g |
| Wollwachs | 4,5 g |
| Campher | 3,0 g |
| Tobramycin (Base) | 0,5 g |
| | 100,0 g |

Die Salbe wird auf die im Beispiel 10 beschriebene Weise zubereitet. Schmelzpunkt: 41-43 °C.

### Beispiel 12

| | |
|---|---|
| Weißes Bienenwachs | 16,0 g |
| Paraffinöl | 76,0 g |
| Wollwachs | 4,5 g |
| Campher | 3,0 g |
| Neomycinsulfat | 0,5 g |

Die Salbe wird auf die im Beispiel 10 beschriebene Weise bereitet. Erstarrungspunkt: 41-43 °C.

### Beispiel 13

| | |
|---|---|
| Weißes Bienenwachs | 16,0 g |
| Paraffinöl | 76,0 g |
| Wollwachs | 4,5 g |
| Campher | 3,0 g |
| Erythromycin-lactobionat | 0,5 g |
| | 100,0 g |

Die Salbe wird auf die im Beispiel 10 beschriebene Weise hergestellt. Erstarrungspunkt: 42-43 °C.

### Beispiel 14

| | |
|---|---|
| Weißes Bienenwachs | 16,0 g |
| Paraffinöl | 76,0 g |
| Wollwachs | 4,5 g |
| Campher | 3,0 g |
| Oxytetracyclin (Base) | 0,5 g |
| | 100,0 g |

Die Salbe wird auf die im Beispiel 10 beschriebene Weise bereitet. Erstarrungspunkt: 42-43 °C.

Beispiel 15

Die gemäß den Beispielen 1-14 hergestellten Salben werden in einer Menge von 1,0-2,0 g auf eine Gazefläche von je 100 cm² ausgestrichen. Die Salbe wird dazu auf 65-70 °C erwärmt.

Beispiel 16

Die gemäß den Beispielen 1-14 hergestellten Salben werden zum Imprägnieren von Textilien verwendet. Dazu wird die Salbe bis kurz über den Erstarrungspunkt erwärmt. Feines, lockeres Mullgewebe wird durch Eintauchen mit der Salbe imprägniert. Das Mullgewebe wird vierfach gefaltet und dann auf die mit Kleber versehene Seite des Pflasters gelegt und angedrückt. Über das auf diese Weise entstandene Kissen wird eine Schutzfolie aufgezogen. Das Pflaster wird in an sich bekannter Weise sterilisiert.

Beispiel 17

Die gemäß den Beispielen 1-15 hergestellten Salben werden zum Imprägnieren von Textilien verwendet. Dazu werden die Salben auf eine Temperatur über dem Erstarrungspunkt erwärmt. Die bereits auf der Klebeseite des Pflasters befestigten Mullkissen werden in einer geeigneten Vorrichtung mit der Salbe bestrichen und dann mit Schutzfolie überzogen. Die Pflaster werden in bekannter Weise verpackt und sterilisiert.

Klinische Fallbeschreibungen

a) Z.S., ein elfjähriger Junge, fiel zu Hause in das auf den Boden geschüttete Kochende Hühnerrupf-Wasser und erlitt am rechten Arm - auf 5 % seiner Körperoberfläche - oberflächliche Verbrennungen zweiten Grades. Die Wunde wurde täglich mit Gaze verbunden, die mit der Salbe gemäß Beispiel 13 imprägniert war. Der Junge war am 12. Tag völlig gesund.

b) F.D., ein Siebzehnjähriger, verbrannte sich zu Hause mit einer Benzinflamme den Handrücken und den Unterarm (rechts), insgesamt auf 3 % seiner Körperoberfläche mit Verbrennungen zweiten Grades. Die Behandlung erfolgte mit Gaze, die mit der Salbe gemäß Beispiel 3 imprägniert war. Die Heilung dauerte 7 Tage. Aus der abgesonderten Wundflüssigkeit ließen sich Staphylococcus aureus Bakterien züchten, jedoch war keine systematische Antibiotikumbehandlung erforderlich.

c) R.F., ein zehnjähriges Mädchen, erlitt zu Hause durch Verbrühen auf 15 % seiner Körperoberfläche Verbrennungen zweiten Grades. Die mit konservativen hautbildenden Mitteln vorgenommene Behandlung war ergebnislos. Nach einer 10tägigen Behandlung mit Gazeverbandszeug, das mit der Salbe gemäß Beispiel 4 imprägniert war, war die Heilung vollständig.

d) G.J., ein 29jähriger Mann, hatte sich 3 Tage vor seiner Einweisung ins Krankenhaus zu Hause durch Verbrühen am linken Unterarm auf 3 % seiner Körperoberfläche Verbrennungen zweiter Grades. Nach seiner Aufnahme ins Krankenhaus erhielt er eine systematische Antibiotikumbehandlung und parallel dazu eine Wundbehandlung mittels in der Salbe gemäß beispiel 3 getränkter Gaze. Nach 7 Tagen trat völlige Heilung ein.

e) L.K,, eine 29jährige Frau, hatte sich 2 Wochen vor ihrer Aufnahme ins Krankenhaus kochendes Wasser über das linke Bein gegossen und dabei auf 1 % ihrer Körperoberfläche Verbrennungen zweiten bis dritten Grades erlitten. Da die konservativen hautbildenden Methoden keinen Erfolg hatten, wurde eine Autotransplantation durchgeführt. Das Transplantationsfeld wurde mit Gaze abgedeckt, die mit der Salbe gemäß Beispiel 6 imprägniert war. Dadurch war eine Antibiotikumbehandlung überflüssig. Die Patientin wurde 7 Tage nach der Operation als geheilt entlassen.

EP 0 308 436 B1

## Tabelle 1

| Behandlung | Anzahl der Tiere | Oberfläche der Wunde $(cm^2)$ | | Unterschied $(cm^2)$ | Kontraktion $(\%)$ |
|---|---|---|---|---|---|
| | | 0. Tag | 8. Tag | | |
| Kontrolle (phys. NaCl-Lsg.) | 6 | $4,34\pm0,32$ | $2,32\pm0,29$ | 2,08 | $46,67\pm6,56$ |
| Referenzen: Mikulitz-Salbe | 18 | $4,99\pm0,71$ | $2,79\pm0,33$ | 2,2 | $43,67\pm5,86$ |
| | | $5,37\pm0,44$ | $2,42\pm0,18$ | 2,95 | $55,00\pm3,85$ |
| | | $5,14\pm0,16$ | $2,36\pm0,20$ | 2,78 | $54,08\pm3,52$ |
| Nebacetin-Salbe | 6 | | | | |
| Braunovidon-Salbe | 6 | $5,49\pm0,52$ | $2,71\pm0,22$ | 2,78 | $50,45\pm2,90$ |
| Beispiel 3 | 18 | $4,93\pm0,33$ | $1,76\pm0,11$ | 3,17 | $64,33\pm3,93$ |
| | | $5,21\pm0,12$ | $1,70\pm0,13$ | 3,51 | $67,39\pm2,47$ |
| | | $5,46\pm0,34$ | $1,62\pm0,12$ | 3,84 | $70,33\pm1,03$ |
| Beispiel 5 | 6 | | | | |
| Beispiel 13 | 6 | $4,84\pm0,16$ | $2,79\pm0,19$ | 2,05 | $42,37\pm2,48$ |
| Beispiel 14 | 6 | | | | |
| Beispiel 15 | 6 | $5,04\pm0,20$ | $3,22\pm0,14$ | 2,82 | $36,06\pm2,04$ |

Mikulitz-Salbe: Silbernitrat
Nebacetin: BYK Gulden, Niederlande
Braunovidon: B.Braun, Melsungen, BRD

## Patentansprüche

**Patentansprüche für folgende Vertragsstaaten : BE, CH, DE, FR, GB, IT, LI, LU, NL, SE**

1. Heilsalbe insbesondere zur Behandlung von Verbrennungen, dadurch gekennzeichnet, daß sie 12-25 Masse% Wachs, 60-88 Masse% pharmakologisch verträgliches Öl, 1-15 Masse% einer kühlenden ätherischen Substanz und gegebenenfalls max. 5 Masse% mit den übrigen Komponenten verträgliche Wirkstoffe enthält.

2. Heilsalbe nach Anspruch 1, dadurch gekennzeichnet, daß sie als Wachs 15-20 Masse% Bienenwachs und/oder Wollwachs, als pharmakologisch verträgliches Öl 68-78 Masse% Paraffinöl, Mygliol, Sonnenblumenöl, Ricinusöl oder deren Gemische, als kühlende ätherische Substanz 2-9 Masse% Campher und/oder Menthol und als Wirkstoff 0,5-2 Masse% Antiseptikum, vorzugsweise Polyvinylpyrrolidin-Jod-Komplex, oder Chemotherapeutikum, vorzugsweise Antibiotika wie Neomycin, Tobramycin, Erythromycin, Oxytetracyclin enthält.

3. Verfahren zur Herstellung einer Heilsalbe zur lokalen Behandlung von Hautschäden, insbesondere Verbrennungen, dadurch gekennzeichnet, daß 12-25 Masse% Wachs, 60-88 Masse% pharmakologisch verträgliches Öl, 1-15 Masse% einer kühlenden ätherischen Substanz und gegebenenfalls max. 5 Masse% mit den übrigen Komponenten verträgliche Wirkstoffe miteinander vermischt, über den Schmelzpunkt erhitzt, homogenisiert und abgekühlt werden.

4. Lokale Applikationsformen zur Behandlung von Hautschäden, insbesondere Verbrennungen, dadurch gekennzeichnet, daß sie auf einen Träger als Textilien wie Mull, Gaze, Leinen, oder aus Folie aufgestrichen die Heilsalbe gemäß Anspruch 1 oder 2 enthalten.

5. Verfahren zur Herstellung von lokalen Applikationsformen, dadurch gekennzeichnet, daß textile Träger wie Mullbinden, Gaze, Leinen, oder Folie mit der Heilsalbe gemäß Anspruch 1 oder 2 bestrichen oder imprägniert werden, wobei auf die Masse des Trägers bezogen das 0,2-10fache, vorzugsweise das 1-3fache an Salbe verwendet und das bestrichene bzw. impregnierte Träger gegebenenfalls steril verpackt wird.

**Patentansprüche für folgende Vertragsstaat : AT**

1. Verfahren zur Herstellung einer Heilsalbe zur lokalen Behandlung von Hautschäden, insbesondere Verbrennungen, dadurch gekennzeichnet, daß 12 - 25 Masse% Wachs, 60 - 88 Masse% pharmakologisch verträgliches Öl, 1 - 15 Masse% einer kühlenden ätherischen Substanz und gegebenenfalls max. 5 Masse% mit den übrigen Komponenten verträgliche Wirkstoffe miteinander vermischt, über den Schmelzpunkt erhitzt, homogenisiert und abgekühlt werden.

2. Verfahren nach Anspruch 1, dadurch gekennzeichnet, daß man als Wachs 15 - 20 Masse% Bienenwachs und/oder Wollwachs, als pharmakologisch verträgliches Öl 68 - 78 Masse% Paraffinöl, Mygliol, Sonnenblumenöl, Ricinusöl oder deren Gemische, als kühlende ätherische Substanz 2 - 9 Masse% Campher und/oder Menthol und als Wirkstoff 0,5 - 2 Masse% Antiseptikum, vorzugsweise Polyvinylpyrrolidin-Jod-Komplex, oder Chemotherapeutikum, vorzugsweise Antibiotika wie Neomycin, Tobramycin, Erythromycin, Oxytetracyclin verwendet.

3. Verfahren zur Herstellung von lokalen Applikationsformen zur Behandlung von Hautschäden, insbesondere Verbrennungen, dadurch gekennzeichnet, daß textile Träger, wie Mullbinden, Gaze oder Leinen, oder Folien mit der Heilsalbe, herstellbar gemäß Anspruch 1 oder 2, bestrichen oder imprägniert werden und gegebenenfalls der bestrichene bzw. imprägnierte Träger steril verpackt wird.

4. Verfahren nach Anspruch 3, dadurch gekennzeichnet, daß auf die Masse des Trägers bezogen das 0,2 - 10fache, vorzugsweise das 1 - 3fache, an Salbe verwendet wird.

## Claims

**Claims for the following Contracting States : BE, CH, DE, FR, GB, IT, LI, LU, NL, SE**

1. Healing ointment, in particular for treating burns, characterized in that it contains 12-25% by wt. wax, 60-88% by wt. of a pharmacologically compatible oil, 1-15% of a cooling ethereal substance and, optionally, max. 5% by wt. of active constituents compatible with the remaining components.

2. Healing ointment according to Claim 1, characterized in that it contains: 15-20% by wt. beeswax and/or wool wax as wax, 68-78% by wt. paraffin oil, mygliol, sunflower oil, castor oil or mixtures thereof as pharmacologically compatible oil, 2-9% camphor and/or menthol as cooling ethereal substance, and 0.5-2% by wt. of an antiseptic, preferably polyvinyl-pyrrolidone-iodine complex, or chemotherapeutics, preferably antibiotics such as Neomycin, Tobramycin, Erythromycin, Oxytetracycline, as active constituents.

3. Process for preparing a healing ointment for topical treatment of skin damage, in particular burns, characterized in that 12-25% by wt. wax, 60-88% by wt. of a pharmacologically compatible oil, 1-15% by wt. of a cooling ethereal substance, and optionally max. 5% by wt. of active constituents compatible with the remaining components are mixed together, heated above the melting point, homogenized and cooled.

4. Topical application forms for treating skin damage, in particular burns, characterized in that they contain the healing ointment according to Claim 1 or 2 coated on a textile carrier such as gauze bandage, gauze, linen or foil.

5. Process for preparing topical application forms, characterized in that textile carriers such as gauze bandages, gauze, linen or foil are coated or impregnated with the healing ointment according to Claim 1 or 2, in which connection the ointment is used in an amount of 0.2-10 times, preferably 1-3 times the mass of the carrier, and the coated or impregnated carrier is optionally packed in a sterile manner.

## Claims for the following Contracting State : AT

1. Process for preparing a healing ointment for topical treatment of skin damage, in particular burns, characterized in that 12-25% by wt. wax, 60 - 88% by wt. of a pharmacologically compatible oil, 1 - 15% by wt. of a cooling ethereal substance, and optionally a maximum of 5% by wt. of active constituents compatible with the remaining components are mixed together, heated above the melting point, homogenized, and cooled.

2. Process according to Claim 1, characterized in that 15 - 20% by wt. of beeswax and/or wool wax is used as wax, 68 - 70% by wt. of paraffin oil, mygliol, sunflower oil, castor oil or mixtures thereof is used as pharmacologically compatible oil, 2 - 9% by wt. of camphor and/or menthol is used as cooling ethereal substance, and 0.5 - 2% by wt. of an antiseptic, preferably polyvinyl-pyrrolidine-iodine complex, or a chemotherapeutic agent, preferably an antibiotic such as Neomycin, Tobramycin, Erythromycin or Oxytetracycline is used as active constituent.

3. Process for preparing topical application forms for treating skin damage, in particular burns, characterized in that textile carriers such as gauze bandages, gauze, linen or films are coated or impregnated with the healing ointment prepared according to Claim 1 or 2, and the coated or impregnated carrier is optionally packed in a sterile manner.

4. Process according to Claim 3, characterized in that the ointment is used in an amount of 0.2 - 10 times, preferably 1 - 3 times, the mass of the carrier.

## Revendications

## Revendications pour les Etats contractants suivants : BE, CH, DE, FR, GB, IT, LI, LU, NL, SE

1. Onguent, en particulier pour le traitement des brûlures, caractérisé en ce qu'il comprend : 12 à 25 % en masse de cire, 60 à 88 % en masse d'huile pharmacologiquement admissible, 1 à 15 % en masse d'une substance éthérée réfrigérante, et le cas échéant, 5 % en masse au plus de principes actifs compatibles avec les autres composés.

2. Onguent selon la revendication 1, caractérisé en ce qu'il comprend : 15 à 20 % en masse de cire telle la cire d'abeille et/ou de laine, 68 à 78 % en masse d'huile pharmacologiquement admissible, telle l'huile de paraffine, l'huile de tournesol, l'huile de ricin, le mygliol et/ou leur mélange, 2 à 9 % en masse de substance éthérée réfrigérante telle le camphre et/ou le menthol, et 0,5 à 2 % de principe actif tel un antiseptique avantageusement le complexe polyvinylpyrrolidine-iode, ou un produit thérapeutique, avantageusement un antibiotique comme la néomycine, la tobramycine, l'érythromycine, l'oxytétracycline.

3. Procédé de préparation d'un onguent pour traiter localement les blessures de la peau comme les brûlures, caractérisé en ce qu'on mélange ensemble : 12 à 25 % en masse de cire, 60 à 88 % en masse d'huile pharmacologiquement admissible, 1 à 15 % en masse d'une substance éthérée réfrigérante, et le cas échéant 5 % en masse au plus de principes actifs compatibles avec les autres composés, on chauffe au-dessus du point de fusion, on homogénéise et on refroidit.

4. Elément d'application locale pour traiter les blessures de la peau, en particulier les brûlures, caractérisé en ce qu'il comprend un support textile tel la mousseline, la gaze ou une feuille, enduit de l'onguent selon la revendication 1 ou 2.

5. Procédé de préparation d'éléments d'application locale, caractérisé en ce qu'on imprègne ou enduit un support textile tel la mousseline ou la gaze, la toile ou des feuilles à l'aide de l'onguent selon la revendication 1 ou 2, où on utilise par rapport à la masse du support recouvert 0,2 à 10 fois, avantageusement 1 à 3 fois d'onguent, et on emballe le cas échéant de manière stérile le support recouvert ou imprégné.

**Revendications pour l'Etat contractant suivant : AT**

1. Procédé pour préparer un onguent pour traiter localement des blessures de la peau, en particulier les brûlures, caractérisé en ce qu'on mélange 12 à 25 % en masse de cire, 60 à 88 % en masse d'une huile pharmacologiquement admissible, 1 à 15 % en masse d'une substance éthérée réfrigérante et le cas échéant 5 % en masse au plus d'un principe actif compatible avec les autres composés, en ce qu'on chauffe la masse au-dessus du point de fusion, on homogénéise et on refroidit.

2. Procédé selon la revendication 1, caractérisé en ce qu'on uutilise 15 à 20 % en masse de cire telle la cire d'abeille et/ou de laine, 68 à 78 % en masse d'huile pharmacologiquement admissible, telle l'huile de paraffine, le mygliol, l'huile de tournesol, l'huile de ricin ou leur mélange, 2 à 9 % en masse d'une substance éthérée réfrigérante, telle le camphre et/ou le menthol, et 0,5 à 2 % en masse de principe actif tel un antiseptique avantageusement le complexe polyvinylpyrrolidine-iode ou un produit thérapeutique, avantageusement un antibiotique tel la néomycine, la tobramycine, l'érythromycine, l'oxytétracycline.

3. Procédé de préparation d'éléments d'application locale pour traiter les blessures de la peau, en particulier les brûlures, caractérisé en ce qu'on imprègne ou on enduit la toile ou des feuilles avec l'onguent préparé selon la revendication 1 ou 2, et en ce qu'on emballe le cas échéant les supports imprégnés ou enduits de manière stérile.

4. Procédé selon la revendication 3, caractérisé en ce qu'on utilise par rapport à la masse du support recouvert 0,2 à 10 fois, avantageusement 1 à 3 fois d'onguent.